# EUROPÄISCHE PATENTANMELDUNG

(11) **EP 1 785 527 A2**
(43) Veröffentlichungstag der Anmeldung: **16.05.2007**
(21) Anmeldenummer: 06123581.8
(22) Anmeldetag: 07.11.2006
(51) Int. Cl.: D21G 9/00

(54) **Verfahren zur Bestimmung einer Festigkeitseigenschaft einer Faserstoffbahn bei deren Herstellung**

(30) Priorität: 15.11.2005 DE 102005054825
(71) Anmelder: Voith Patent GmbH, 89522 Heidenheim (DE)
(72) Erfinder: Schmachtel, Rainer, Dr., 89522 Heidenheimn (DE); Koester, Soeren, Dr., 89522 Heidenheim (DE); Muench, Rudolf, Dr., 89551 Koenigsbronn (DE)

(57) **Zusammenfassung**

Die Erfindung betrifft ein Verfahren zur Bestimmung zumindest einer Festigkeitseigenschaft einer Faserstoffbahn bei deren Herstellung mit folgenden Schritten:
- Ermittlung des Bahnzugs sowie der daraus resultierenden Spannung der Faserstoffbahn,
- Ermittlung des Ist-Zustandes zumindest einer die Festigkeitseigenschaft der Faserstoffbahn beeinflussenden Regelgröße, und
- Bestimmung des Ist-Zustandes der zumindest einen Festigkeitseigenschaft der Faserstoffbahn mittels eines Modells, welches eine Abhängigkeit der Festigkeitseigenschaft vom Bahnzug, von der Bahnspannung und der zumindest einen Regelgröße herstellt.

## Beschreibung

Die Erfindung betrifft ein Verfahren zur Bestimmung zumindest einer Festigkeitseigenschaft einer Faserstoffbahn bei deren Herstellung, wobei es sich bei der Faserstoffbahn insbesondere um eine Papier-, Karton- oder Tissuebahn handelt.

Mit den aus dem Stand der Technik bekannten Verfahren können Festigkeitseigenschaften von Papier, wie beispielsweise Spaltbarkeit, Bruchdehnung oder Tensile, bisher nur offline, das heißt nach Probenentnahme im Labor ermittelt werden.

Dies hat den Nachteil, dass eine unmittelbare Reaktion während der laufenden Produktion auf Schwankungen der Bahnfestigkeit außerhalb des Normbereichs nicht möglich ist, was aufgrund der dadurch bedingten Zeitverzögerung zu hoher Ausschussproduktion führen kann.

Des Weiteren sind die Labormessungen zeitaufwändig und daher kostenintensiv.

Es ist die Aufgabe der vorliegenden Erfindung, ein Verfahren der eingangs geannten Art vorzuschlagen, bei dem die beschriebenen Nachteile nicht mehr auftreten.

Die Aufgabe wird erfindungsgemäß durch die Merkmale des Patentanspruchs 1 gelöst.

Erfindungsgemäß wird ein Verfahren zur Bestimmung zumindest einer Festigkeitseigenschaft einer Faserstoffbahn bei deren Herstellung mit folgenden Schritten vorgeschlagen:
- Ermittlung des Bahnzugs sowie der daraus resultierenden Spannung der Faserstoffbahn,
- Ermittlung des Ist-Zustandes zumindest einer die Festigkeitseigenschaft der Faserstoffbahn beeinflussenden Regelgröße, und
- Bestimmung des Ist-Zustandes der zumindest einen Festigkeitseigenschaft der Faserstoffbahn mittels eines Modells, welches eine Abhängigkeit der Festigkeitseigenschaft vom Bahnzug, von der Bahnspannung und der zumindest einen Regelgröße herstellt.

Das erfindungsgemäße Verfahren beruht auf der Idee, die zumindest eine Festigkeitseigenschaft indirekt mittels eines geeigneten Modells zu ermitteln, welches direkt messbare Eigenschaften der Faserstoffbahn und beim Herstellungsprozess beeinflussbare Regelgrößen berücksichtigt. Hierdurch muss nicht mehr wie aus dem Stand der Technik bekannt, ein Festigkeitswert direkt gemessen werden, was bis heute nur im Labor möglich ist.

Das erfindungsgemäße Verfahren schlägt vielmehr vor, zur indirekten Bestimmung einer Festigkeitseigenschaft den Bahnzug und die daraus resultierende Bahnspannung zu ermitteln. Dies ist besonders vorteilhaft, da bereits heute bei Papiermaschinen der Bahnzug und die daraus resultierende Bahnspannung ermittelt werden, um beispielsweise beim Aufwickeln der Papierbahn die geeignete Wickelhärte und eine fehlerfreie Aufwicklung zu regeln. Daher kann mit dem erfindungsgemäßen Verfahren auf bereits in der Papiermaschine installierte Sensoren zurückgegriffen werden.

Vorteilhafte Ausführungsformen und Weiterbildungen der Erfindung sind in den Unteransprüchen angegeben.

Es ist insbesondere sinnvoll, wenn auf Basis des bestimmten Ist-Wertes der zumindest einen Festigkeitseigenschaft diese bei der Herstellung der Faserstoffbahn geregelt wird. Hierzu sieht eine besonders bevorzugte Ausgestaltung der Erfindung folgende weiteren Verfahrensschritte vor:
- Vergleich des Ist-Zustandes der zumindest einen Festigkeitseigenschaft mit dem Soll-Zustand der zumindest einen Festigkeitseigenschaft,
- Veränderung des Ist-Zustandes der zumindest einen Regelgröße zur Einstellung des Soll-Zustandes der zumindest einen Festigkeitseigenschaft.

Somit wird durch die Erfindung eine automatische Maschinenregelung geschaffen, welche in der Lage ist, bei laufender Produktion die Regelgrößen beziehungsweise Prozessparameter hinsichtlich der Festigkeit der Faserstoffbahn zu optimieren.

Konkret kann die zumindest eine Festigkeitseigenschaft der Faserstoffbahn umfassen: deren Spaltbarkeit, deren Längs- und/oder Querbruchdehnbarkeit, deren Tensile, deren Reißlänge.

Vorzugsweise werden der Bahnzug und die daraus resultierende Bahnspannung im Bereich des Aufwickelns bestimmt. Hierdurch können die zur Regelung des Aufwickelprozesses in der Papiermaschine vorhandenen Sensoren verwendet werden.

Um eine kontinuierliche Regelung der zumindest einen Festigkeitseigenschaft während des Herstellungsprozesses sicherzustellen, wird der Bahnzug und die Bahnspannung sowie der Ist-Zustand der zumindest einen Regelgröße vorzugsweise online bestimmt. Bei der online Bestimmung können der Bahnzug und die Bahnspannung und/oder die zumindest eine Regelgröße beispielsweise kontinuierlich oder in vorgegebenen Zeitabständen ermittelt werden.

Bei der Aufstellung des Modells werden vorzugsweise gemessene Werte der zumindest einen Festigkeitseigenschaft berücksichtigt, um einen Vergleich zwischen den indirekt unter Verwendung des Bahnzugs und der Bahnspannung ermittelten Werten der zumindest einen Festigkeitseigenschaft und den direkt aus der Faserstoffbahn ermittelten Werten der zumindest einen Festigkeitseigenschaft herzustellen, wodurch gewährleistet wird, dass das verwendete Modell den Anforderungen genügt. Bei dem Modell kann es sich insbesondere um ein selbst lernendes Modell handeln, welches bei Abweichungen zwischen indirekt und direkt ermittelten Werten der zumindest einen Festigkeitseigenschaft selbstständig eine entsprechende Parameteranpassung vornimmt.

Vorzugsweise wird die zumindest eine Festigkeitseigenschaft an einer Probe der Faserstoffbahn gemessen, welche der fertig produzierten Faserstoffbahn, beispielsweise vom fertig gewickelten Tambour, entnommen wurde. Die direkt gemessenen Werte der zumindest einen Festigkeitseigenschaft werden hierbei vorzugsweise im Labor ermittelt.

Des Weiteren können gemessene Werte der zumindest einen Festigkeitseigenschaft auch zur laufenden beziehungsweise regelmäßigen Kalibrierung der den Bahnzug und die Bahnspannung messenden Sensoren verwendet werden.

Das im erfindungsgemäßen Verfahren verwendete Modell berücksichtigt vorzugsweise zusätzlich zum Bahnzug, der Bahnspannung und dem Ist-Zustand der zumindest einen Regelgröße die Sorte der Faserstoffbahn und/oder spezifische Eigenarten des verwendeten Herstellungsprozesses und/oder die Bauart der die Faserstoffbahn herstellenden Maschine, wodurch das Modell weiter verfeinert wird.

Durch die weitere Verfeinerung wird erreicht, dass der mit dem Modell indirekt ermittelte Ist-Zustand der zumindest einen Festigkeitseigenschaft sehr genau dem realen Wert des Ist-Zustandes der zumindest einen Festigkeitseigenschaft entspricht, wie dieser mit der direkten Messung im Labor ermittelt werden kann.

Die Festigkeit der Faserstoffbahn wird beispielsweise durch die Zusammensetzung der Stoffsuspension, das heißt beispielsweise durch die chemischen Zusätze, den oder die verwendeten Faserstoffe, bei mehreren Faserstoffen durch deren Verhältnis zueinander oder durch den Füllstoffanteil mitbestimmt. Eine bevorzugte Ausgestaltung sieht demzufolge, vor dass die zumindest eine Regelgröße die Zusammensetzung der für die Herstellung der Faserstoffbahn verwendeten Stoffsuspension umfasst.

Eine zur obigen zusätzliche oder alternative Ausgestaltung der Erfindung sieht vor, dass die zumindest eine Regelgröße das Verhältnis von Bahnzügen an verschiedenen Stellen des Herstellungsprozesses der Faserstoffbahn umfasst. So wird beispielsweise die Spaltbarkeit der Faserstoffbahn entscheidend mit durch das Verhältnis des Bahnzugs vor dem letzten Pressnip der Pressenpartie zum Bahnzug nach dem letzten Pressnip der Pressenpartie bestimmt.

Selbstverständlich ist es mit dem erfindungsgemäßen Verfahren auch möglich, dass das Modell mehrere Regelgrößen berücksichtigt.

Eine weitere besonders bevorzugte Ausgestaltung der Erfindung sieht, vor dass ein Querprofil der zumindest einen Festigkeitseigenschaft ermittelt wird, indem die Faserstoffbahn in mehrere Abschnitte geschnitten wird, die sich jeweils über eine Teilbreite der Faserstoffbahn erstrecken und indem anschließend für jeden Abschnitt der Bahnzug und die daraus resultierende Bahnspannung ermittelt wird.

Die Bestimmung des Querprofils der zumindest einen Festigkeitseigenschaft ist beispielsweise vorzugsweise in einer Rollenschneidmaschine möglich. Hierbei wird der maschinenbreite Tambour in mehrere schmale Papierrollen zerteilt. Durch Bahnzug- und Bahnspannungsmessung an jeder einzelnen Rolle verbunden mit der Auswertung mittels des erfindungsgemäßen Modells lässt sich somit ohne direkte Labormessung die zumindest eine Festigkeitseigenschaft während der laufenden Produktion bestimmen und regeln.

Nachfolgend wird die Erfindung anhand eines Ausführungsbeispiels durch die folgende Zeichnung weiter erläutert.

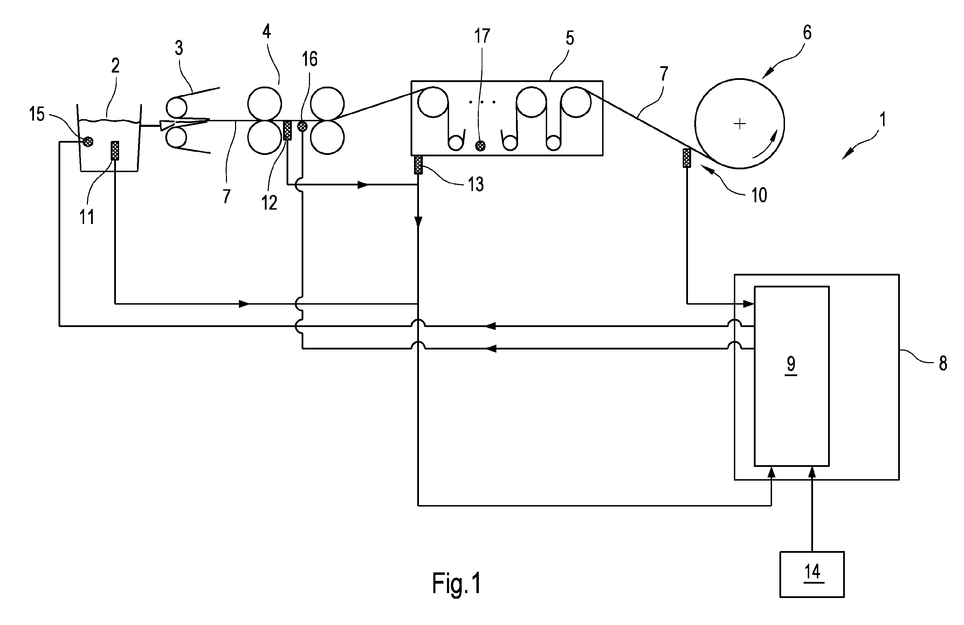

Es zeigt
- Figur 1: eine Maschine, die zur Durchführung des erfindungsgemäßen Verfahrens geeignet ist, in schematischer Darstellung.

Die Figur 1 zeigt eine Papiermaschine 1 mit einer Stoffaufbereitung 2, einer Formierpartie 3, einer Pressenpartie 4, einer Trockenpartie 5 und einer Aufwickeleinrichtung 6 zur Aufwicklung einer fertig produzierten Papierbahn 7.

Des Weiteren weist die Papiermaschine 1 eine Regelungseinheit 8 zur Regelung des Papierherstellungsprozesses auf. Die Regelungseinheit 8 umfasst eine Untereinheit 9, mit welcher die zumindest eine Festigkeitseigenschaft, im konkreten Fall die Spaltbarkeit der Papierbahn 7, während des laufenden Produktionsprozesses geregelt wird.

Im Bereich der Aufwickeleinrichtung 6 ist ein Sensor 10 vorgesehen, mit welchem der Bahnzug und die daraus resultierende Bahnspannung der Papierbahn 7 ermittelt werden kann.

Gemäß dem erfindungsgemäßen Verfahren führt die Untereinheit 9 zur Regelung der Spaltbarkeit der Papierbahn 7 die folgenden Schritte durch. Mittels des Sensors 10 wird der Bahnzug sowie die daraus resultierende Spannung der Papierbahn 7 ermittelt. Des Weiteren wird mittels Sensoren 11 der Ist-Zustand der Faserstoffzusammensetzung in der Stoffaufbereitung 2 sowie mittels Sensoren 12 und 13 der Ist-Zustand des Verhältnisses des Bahnzugs vor dem letzten Pressnip der Pressenpartie 4 zum Bahnzug nach dem letzten Pressnip der Pressenpartie 4 ermittelt.

Die so erhaltenen Daten werden der Untereinheit 8 zugeführt, welche auf Basis dieser Daten sowie unter Berücksichtigung von weiteren von einer Prozessdatenüberwachungseinheit 14 bereitgestellten Prozessdaten und beispielsweise unter Berücksichtigung der Sorte der Papierbahn 7 und/oder spezifischer Eigenarten des verwendeten Herstellungsprozesses und/oder der Bauart der Papiermaschine 1 mittels eines Modells die Spaltbarkeit der Papierbahn 7 bestimmt. Wie bereits erwähnt, kann durch die Untereinheit 9 auch eine Kalibrierung der Sensoren 11 bis 13 auf Basis von Spaltbarkeitswerten durchgeführt werden, welche im Labor an der fertig produzierten Papierbahn 7 ermittelt wurden.

Anschließend wird durch die Untereinheit 9 der Ist-Zustand der Spaltbarkeit mit dem Soll-Zustand der Spaltbarkeit verglichen und darauf basierend von der Untereinheit 9 Aktuatoren 15, 16 und 17 angesteuert, um den Ist-Zustandes der Spaltbarkeit auf den Soll-Zustand einzustellen, indem der Ist-Zustand der Regelgrößen Faserstoffzusammensetzung in der Stoffaufbereitung 2 und Verhältnis des Bahnzugs vor dem letzten Pressnip der Pressenpartie 4 zum Bahnzug nach dem letzten Pressnip der Pressenpartie 4 entsprechend verändert wird.

Bei der dargestellten Ausführungsform werden der Bahnzug und die Bahnspannung beim Aufwickeln sowie die Regelgrößen online und zwar kontinuierlich bestimmt.

## Patentansprüche

1. Verfahren zur Bestimmung zumindest einer Festigkeitseigenschaft einer Faserstoffbahn bei deren Herstellung mit folgenden Schritten:
- Ermittlung des Bahnzugs sowie der daraus resultierenden Spannung der Faserstoffbahn,
- Ermittlung des Ist-Zustandes zumindest einer die Festigkeitseigenschaft der Faserstoffbahn beeinflussenden Regelgröße, und
- Bestimmung des Ist-Zustandes der zumindest einen Festigkeitseigenschaft der Faserstoffbahn mittels eines Modells, welches eine Abhängigkeit der Festigkeitseigenschaft vom Bahnzug, von der Bahnspannung und der zumindest einen Regelgröße herstellt.

2. Verfahren nach Anspruch 1,
**dadurch gekennzeichnet,**
**dass** zur Regelung der zumindest einen Festigkeitseigenschaft die folgenden weiteren Verfahrensschritte vorgesehen sind:
- Vergleich des Ist-Zustandes der zumindest einen Festigkeitseigenschaft mit dem Soll-Zustand der zumindest einen Festigkeitseigenschaft, und
- Veränderung des Ist-Zustandes der zumindest einen Regelgröße zur Einstellung des Soll-Zustandes der zumindest einen Festigkeitseigenschaft.

3. Verfahren nach Anspruch 1 oder 2,
**dadurch gekennzeichnet,**
**dass** der Bahnzug und die daraus resultierende Bahnspannung im Bereich des Aufwickelns bestimmt werden.

4. Verfahren nach einem der vorhergehenden Ansprüche,
**dadurch gekennzeichnet,**
**dass** das Modell zusätzlich die Sorte der Faserstoffbahn und/oder spezifische Eigenarten des verwendeten Herstellungsprozesses und/oder die Bauart der die Faserstoffbahn herstellenden Maschine berücksichtigt.

5. Verfahren nach einem der vorhergehenden Ansprüche,
**dadurch gekennzeichnet,**
**dass** der Bahnzug und die Bahnspannung sowie die zumindest eine Regelgröße online bestimmt werden.

6. Verfahren nach einem der vorhergehenden Ansprüche,
**dadurch gekennzeichnet,**
**dass** bei der Aufstellung des Modells gemessene Werte der zumindest einen Festigkeitseigenschaft berücksichtigt werden.

7. Verfahren nach Anspruch 6,
**dadurch gekennzeichnet,**
**dass** die gemessenen Werte im Labor ermittelt werden.

8. Verfahren nach einem der vorhergehenden Ansprüche,
**dadurch gekennzeichnet,**
**dass** das Modell mehrere Regelgrößen berücksichtigt.

9. Verfahren nach einem der vorhergehenden Ansprüche,
**dadurch gekennzeichnet,**
**dass** die zumindest eine Regelgröße die Zusammensetzung der für die Herstellung der Faserstoffbahn verwendeten Stoffsuspension, insbesondere des Faserstoffs umfasst.

10. Verfahren nach einem der vorhergehenden Ansprüche,
**dadurch gekennzeichnet,**
**dass** die zumindest eine Regelgröße das Verhältnis von Bahnzügen an verschiedenen Stellen des Herstellungsprozesses der Faserstoffbahn ist.

11. Verfahren nach Anspruche 10,
**dadurch gekennzeichnet,**
**dass** die zumindest eine Regelgröße das Verhältnis eines Bahnzugs vor zu einem Bahnzug nach dem letzten Pressnip der Pressenpartie ist.

12. Verfahren nach einem der vorhergehenden Ansprüche,
**dadurch gekennzeichnet,**
**dass** die zumindest eine Festigkeitseigenschaft der Faserstoffbahn umfasst: deren Spaltbarkeit, deren Längs- und/oder Querbruchdehnbarkeit, deren Tensile, deren Reißlänge.

13. Verfahren nach einem der vorhergehenden Ansprüche,
**dadurch gekennzeichnet,**
**dass** ein Querprofil der zumindest einen Festigkeitseigenschaft ermittelt wird, indem die Faserstoffbahn in mehrere Abschnitte geschnitten wird, die sich jeweils über eine Teilbreite der Faserstoffbahn erstrecken und indem anschließend für jeden Abschnitt der Bahnzug und die daraus resultierende Bahnspannung ermittelt werden.

14. Verfahren nach Anspruche 13,
**dadurch gekennzeichnet,**
**dass** das Querprofil der zumindest einen Festigkeitseigenschaft im Bereich des Rollenschneidens ermittelt wird.
